# EUROPEAN PATENT SPECIFICATION

(11) **EP 3 943 133 B1**
(45) Date of publication and mention of the grant of the patent: **13.11.2024**
(21) Application number: 20783495.3
(22) Date of filing: 17.03.2020
(51) Int. Cl.: A61M 5/00, A61M 5/32, A61M 5/315

(54) **PLUNGER KIT, SYRINGE KIT, AND PREFILLED SYRINGE**
KOLBENSET, SPRITZENSET UND FERTIGSPRITZE
KIT DE PISTON, KIT DE SERINGUE, ET SERINGUE PRÉ-REMPLIE

(30) Priority: 29.03.2019 JP 2019065682
(43) Date of publication of application: 26.01.2022
(60) Divisional application: 24204911.2
(73) Proprietor: TERUMO Kabushiki Kaisha, Tokyo 151-0072 (JP)
(72) Inventor: TAKEMOTO, Masafumi, Fujinomiya-shi, Shizuoka 418-0004 (JP); FUKE, Shigeaki, Fujinomiya-shi, Shizuoka 418-0004 (JP); HIGUCHI, Yasuaki, Tokyo 163-1450 (JP); GOTO, Kazunori, Fujinomiya-shi, Shizuoka 418-0004 (JP)
(74) Representative: Casalonga
(86) International application number: PCT/JP2020/011599
(87) International publication number: WO 2020/203239

(56) References cited:
- EP-A1- 1 459 775
- WO-A1-01/97885
- WO-A1-2012/124028
- WO-A1-2012/134430
- WO-A1-2013/115331
- WO-A1-2018/180758
- JP-A- 2003 199 827
- JP-A- 2004 283 466
- JP-A- 2011 254 891
- JP-A- 2012 045 203
- JP-A- 2016 187 530
- JP-A- 2017 148 554
- JP-A- 2018 158 179
- JP-A- 2019 501 735
- JP-B2- S5 824 200
- JP-B2- S6 349 661
- US-A1- 2012 136 298

## Description

### Technical Field

The present invention relates to a plunger kit, a syringe kit, and a prefilled syringe. In particular, the present invention relates to a plunger kit according to the preamble of claim 1, such as it is e.g. known from JP 2012 045203 A.

### Background Art

A prefilled syringe used for injecting liquid medicine includes a syringe main body including a liquid medicine storage chamber, liquid medicine filled in the liquid medicine storage chamber, a cap attached to a nozzle of the syringe main body, a piston (also referred to as a piston or a gasket) slidably fitted inside of the syringe main body, and a plunger rod for sliding the piston in an axial direction. Such a prefilled syringe is assembled by filling the liquid medicine inside the syringe main body with the cap attached to the nozzle, capping only the piston first to seal the liquid medicine in the syringe main body, and pushing a tip end part of the plunger rod into a recess of a base end side of the capped piston to join the plunger rod and the piston.

Incidentally, when a plunger is coupled to the piston, it is necessary to push the plunger so as to press the plunger toward a tip end side of the syringe. At this time, there is a possibility that a problem, such as the piston moves and liquid medicine leaks from the syringe, arises.

In order to solve such a problem, WO 2009/011214 A discloses a syringe plunger assembly for injector in which a cylindrical hole opened on a base end side is provided in a piston, and a male screw structure is provided at a tip end part of a plunger rod.

### Summary of Invention

However, in a technique of WO 2009/011214 A, the male screw structure having an outer diameter larger than an inner diameter thereof is pushed into a hole of the piston, and the male screw structure is caused to bite into an inner wall of the hole, thereby coupling the plunger rod with the piston. Therefore, in order to prevent the plunger rod from falling off from the piston during transportation or the like, it is necessary that an outer diameter of the male screw structure of the plunger rod is sufficiently larger than an inner diameter of the hole of the piston. However, in this case, there is a problem that the piston moves because the plunger rod is forcibly pushed into the hole of the piston at the time of coupling.

An object of the present embodiment is to provide a plunger kit, a syringe kit, and a prefilled syringe capable of ensuring joint strength between a plunger rod and a piston, enabling mounting of the plunger rod to the piston without moving the piston, and preventing the plunger rod from falling off from the piston during transportation, or the like, of the prefilled syringe.

The invention also provides a syringe kit including the plunger kit of the above-described aspect, and a syringe main body part having a liquid medicine storage chamber that stores liquid medicine, a nozzle provided on a tip end of the liquid medicine storage chamber, and a cap that seals a tip end opening part of the nozzle, in which the piston is slidable in the liquid medicine storage chamber.

Still another aspect of the present disclosure is a prefilled syringe including the syringe kit of the above-described aspect, and liquid medicine filled in the liquid medicine storage chamber, in which the piston is disposed so as to be slidable in the liquid medicine storage chamber.

According to the plunger kit, syringe kit, and prefilled syringe in the above-described aspects, the plunger rod can be mounted to the piston without moving the piston, and the piston does not come out of the plunger during transportation, or the like, of the prefilled syringe.

### Brief Description of Drawings

Fig. 1 is a perspective view that illustrates a syringe kit according to a first embodiment, the syringe kit being with a plunger rod detached.
Fig. 2A is a cross-sectional view of a syringe main body part in Fig. 1, Fig. 2B is a perspective view of the piston in Fig. 1, and Fig. 2C is a cross-sectional view of the piston in Fig. 2B.
Fig. 3 is a perspective view of a rod main body and mounting part of the plunger rod in Fig. 1.
Fig. 4A is a side view of the rod main body and mounting part of the plunger rod in Fig. 3, Fig. 4B is similarly a top view, and Fig. 4C is a front view of the mounting part in Fig. 4A.
Fig. 5A is a cross-sectional view that illustrates a state in a process of mounting the mounting part of the plunger rod to the piston as viewed from an upper surface side, Fig. 5B is a cross-sectional view that illustrates similarly a state in a process of mounting the mounting part to the piston as viewed from a side surface direction, and Fig. 5C is a cross-sectional view that illustrates the plunger kit, syringe kit, and prefilled syringe according to the first embodiment.
Fig. 6 is a cross-sectional view that illustrates the syringe kit of the first embodiment in an assembled state.
Fig. 7 is a perspective view that illustrates another aspect of the syringe kit of the first embodiment.
Fig. 8A is a front view of a mounting part of a plunger rod according to a second embodiment, Fig. 8B is a side view of the mounting part and main body part of the plunger rod according to the second embodiment, and Fig. 8C is a cross-sectional view along the line VIIIC-VIIIC in Fig. 8B.
Fig. 9A is a cross-sectional view that illustrates a process of inserting the mounting part in Fig. 8B to the piston, Fig. 9B is a cross-sectional view that illustrates a state of the mounting part in Fig. 8A passing through a narrow part of the piston, and Fig. 9C is a cross-sectional view that illustrates the plunger kit, syringe kit, and prefilled syringe according to the second embodiment.
Fig. 10A is a perspective view of a mounting part of a plunger rod according to a third embodiment, and Fig. 10B is similarly a front view of the mounting part of the plunger rod.
Fig. 11A is a cross-sectional view that illustrates a state in a process of mounting the mounting part of the plunger rod in Fig. 10A to the piston as viewed from an upper surface side, and Fig. 11B is a cross-sectional view that illustrates a state in a process of mounting the mounting part of the plunger in Fig. 10A to the piston as viewed from a side surface direction.
Fig. 12 is a cross-sectional view that illustrates the plunger kit, syringe kit, and prefilled syringe according to the third embodiment.
Fig. 13A is a partially cutaway cross-sectional view of a plunger rod according to a fourth embodiment, and Fig. 13B is a perspective view of the plunger rod according to the fourth embodiment.
Fig. 14 is an explanatory diagram that illustrates a method for forming coating for the plunger rod according to the fourth embodiment.
Fig. 15 is a cross-sectional view of a piston according to a fifth embodiment.
Fig. 16A is an explanatory diagram that illustrates a first method for forming coating for the piston illustrated in Fig. 15, Fig. 16B is an explanatory diagram that illustrates a second method for forming coating for the piston illustrated in Fig. 15, and Fig. 16C is an explanatory diagram that illustrates a third method for forming coating for the piston according to Fig. 15.
Fig. 17A is a side view of vicinity of a mounting part of a plunger rod according to a sixth embodiment, Fig. 17B is a front view of the mounting part in Fig. 17A, and Fig. 17C is a cross-sectional view that illustrates the plunger kit, syringe kit, and prefilled syringe according to the sixth embodiment.

### Description of Embodiments

Hereinafter, preferred embodiments of the present invention will be described in detail with reference to the appended drawings. In addition, in the following description, a direction in which a nozzle 12c of a syringe kit 10 is formed is referred to as a tip end side or a tip end direction, and a direction opposite to the nozzle 12c of the syringe kit 10 is referred to as a base end side or a base end direction.

### (First embodiment)

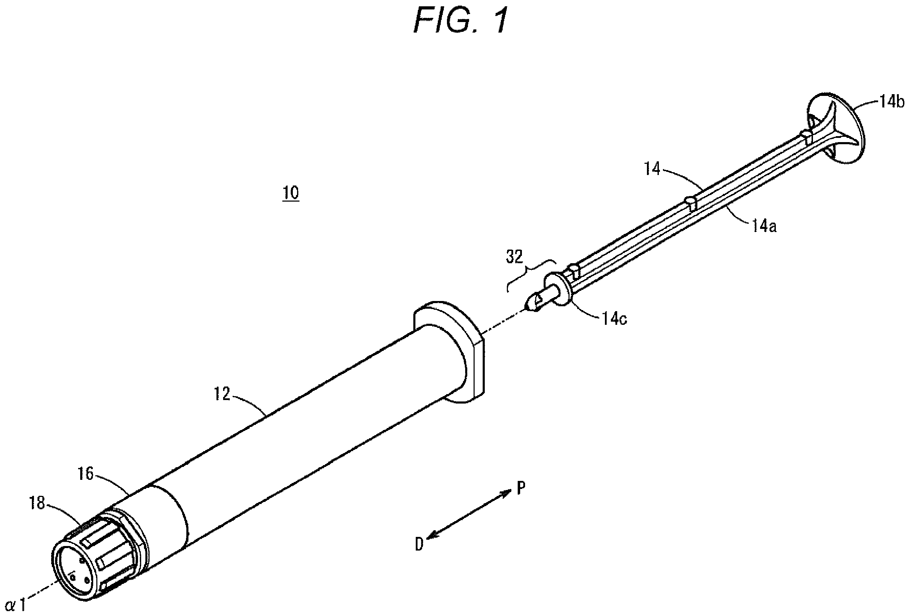

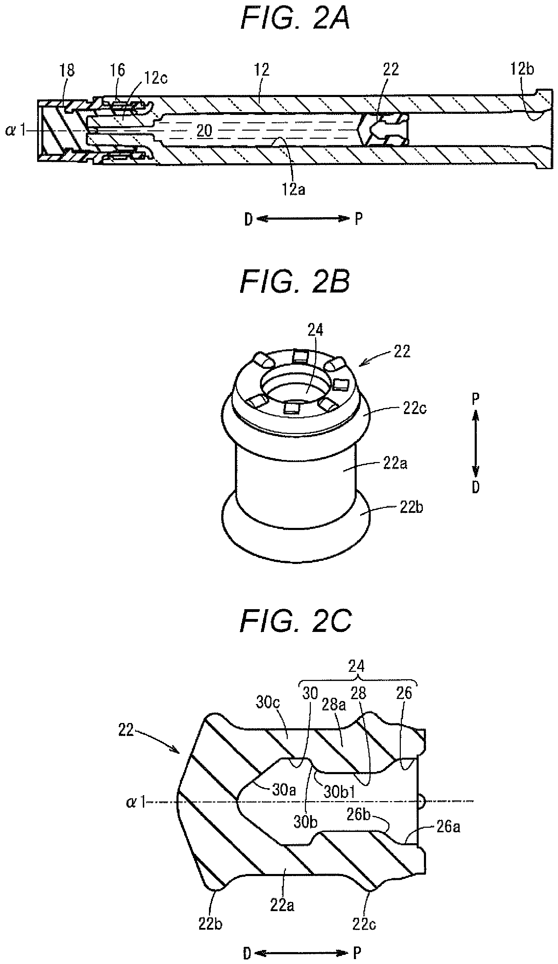

As illustrated in Fig. 1, a syringe kit 10 according to the present embodiment includes a syringe main body part 12 and a plunger rod 14. The syringe main body part 12 is not particularly limited, but is, for example, a prefilled syringe 100 including liquid medicine 20 sealed inside. As illustrated in Fig. 2A, the syringe main body part 12 is formed in a cylindrical shape, and a liquid medicine storage chamber 12a is formed therein extending in an axial direction. A base end part 12b of the liquid medicine storage chamber 12a is opened, and is configured to allow insertion of the plunger rod 14.

A nozzle 12c is formed at a tip end of the liquid medicine storage chamber 12a, and a connector 16 is attached to an outer peripheral part of the nozzle 12c. A cap 18 is screwed to the connector 16, and a tip end opening part of the nozzle 12c is sealed by the cap 18. That is, a tip end side of the liquid medicine storage chamber 12a is sealed by the cap 18. A piston 22 is inserted into the liquid medicine storage chamber 12a slidably in the axial direction. A base end side of the liquid medicine storage chamber 12a is sealed by the piston 22.

As illustrated in Fig. 1, during transportation and storage, the syringe kit 10 is provided with the plunger rod 14 detached. The plunger rod 14 is inserted from the base end part 12b of the syringe main body part 12 immediately before use and mounted to the piston 22. It should be noted that the syringe kit 10 may be provided with the plunger rod 14 mounted to the piston 22 in advance. Hereinafter, a coupling structure of the piston 22 and plunger rod 14 will be described in more detail.

The piston 22 includes, for example, a medical rubber material, and includes a main body part 22a having a columnar shape, ring-shaped protrusions 22b, 22c formed on side portions of the main body part 22a, and a recess 24 as illustrated in Fig. 2B. The main body part 22a is formed to be smaller than an inner diameter of the liquid medicine storage chamber 12a, while the ring-shaped protrusions 22b, 22c are formed to be larger than the inner diameter of the liquid medicine storage chamber 12a. In the syringe main body part 12, the ring-shaped protrusions 22b, 22c abut against and adhere tightly to an inner wall of the liquid medicine storage chamber 12a, thereby sealing the liquid medicine storage chamber 12a. A recess 24 having a substantially circular cross section as viewed from a base end side is formed inside the piston 22. The recess 24 is open on a base end side of the piston 22.

As illustrated in Fig. 2C, the recess 24 includes an opening part 26 formed on the base end side of the piston 22, a narrow part 28 communicating with a tip end side of the opening part 26, and a storage part 30 communicating with a tip end side of the narrow part 28. The narrow part 28 is formed on an inner side of a second side wall part 28a at which thickness of the piston 22 in a radial direction is increased, and is formed such that length of the second side wall part 28a in the axial direction is 10% to 40% of an entire length of the piston 22 in the axial direction. By setting length of the second side wall part 28a forming the narrow part 28 to such a length, the narrow part 28 is less likely to expand with respect to input of load via the plunger rod 14. Therefore, even if force toward the base end direction acts on the plunger rod 14 during transportation or the like, a problem of the plunger rod 14 falling off from the piston 22 can be prevented. It should be noted that the length of the second side wall part 28a in the axial direction is preferably 10% to 30% of the entire length of the piston 22 in the axial direction.

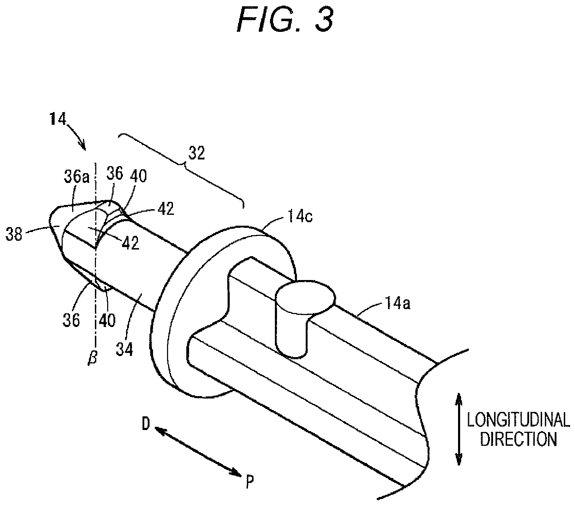

The narrow part 28 formed on the inner side of the second side wall part 28a is reduced in diameter to be smaller than the opening part 26. The narrow part 28 is formed to have a substantially constant inner diameter and extend in the axial direction by the same length as the second side wall part 28a. By setting the narrow part 28 to have a constant inner diameter in this manner, a mounting part 32 can smoothly pass through the narrow part 28. In addition, the inner diameter of the narrow part 28 is slightly larger than an outer diameter of a shaft part 34 (refer to Fig. 3) of the plunger rod 14 described later.

The narrow part 28 is formed such that length thereof in the axial direction is 10% to 40% of length of the piston 22 in the axial direction. Specifically, the length of the narrow part 28 can be set to about 1 mm to 5 mm. By being formed in this manner, the narrow part 28 of the piston 22 is less likely to be deformed by tensile force when the plunger rod 14 is pulled out, and sufficient joint strength can be exerted against the tensile force. It should be noted that the length of the narrow part 28 is preferably 1 mm to 3 mm.

In addition, a ring-shaped protrusion 22c is provided on an outer peripheral part of the second side wall part 28a that constitutes the narrow part 28. By providing the ring-shaped protrusion 22c on the outer peripheral part of the second side wall part 28a in this manner, the ring-shaped protrusion 22c is pressed against the liquid medicine storage chamber 12a when the mounting part 32 of the plunger rod 14 passes, and therefore the piston 22 is less likely to move, and displacement of the piston 22 can be prevented.

The storage part 30 is formed on an inner side of a first side wall part 30c. The first side wall part 30c is formed on a tip end side of the second side wall part 28a, and is thinner in the radial direction than the second side wall part 28a. At a boundary between the first side wall part 30c and the second side wall part 28a, an abutting surface 30b1 is formed so as to rise substantially perpendicular to the axial direction.

The storage part 30 formed on the inner side of the first side wall part 30c includes a base part 30b formed to have an inner diameter substantially equal to an inner diameter of the opening part 26, and a reduced diameter part 30a formed on a tip end side of the base part 30b and reducing in diameter in a conical shape toward the tip end. The base part 30b is formed to have an inner diameter larger than engagement parts 36 (refer to Fig. 3) of the plunger rod 14, the engagement parts 36 being described later, and is configured to be able to store the engagement parts 36. At a boundary part between the base part 30b and the narrow part 28, the abutting surface 30b1 is formed so as to rise substantially perpendicular to the axial direction. The abutting surface 30b1 is configured to exert resistance force against pulling out, by abutting against an engagement surface 40 (refer to Fig. 3) formed on a base end side of the engagement parts 36. The reduced diameter part 30a is a part that stores a conical part 38 (refer to Fig. 3), and is configured by a conical surface inclined at substantially the same angle as the conical part 38.

The opening part 26 has a base end part 26a formed to have an inner diameter substantially the same as an outer diameter of the engagement parts 36 along an axis α1 direction and extending in the axis α1 direction by a predetermined length, and a tapered part 26b reduced in diameter from the base end part 26a toward the narrow part 28. The base end part 26a is configured to guide an axis of the engagement parts 36 of the plunger rod 14 to an axis α1 of the recess 24, the engagement parts 36 being formed in a tapered shape. The tapered part 26b guides the conical part 38 to a center of the axis α1 of the recess 24. With this arrangement, the axes of the plunger rod 14 and piston 22 are aligned, and the engagement parts 36 of the mounting part 32 easily pass through the narrow part 28.

As illustrated in Fig. 1, the plunger rod 14 includes a pressing part 14b to which load toward a forward direction or a backward direction is input, a rod main body 14a that is formed to extend in the axial direction and transmits the input load, and a mounting part 32 formed on a tip end side of the rod main body 14a. As illustrated in Fig. 3, a piston abutting part 14c having a disk shape and perpendicular to the axial direction is formed on the tip end side of the rod main body 14a. The mounting part 32 is a part formed to extend toward the tip end side from the piston abutting part 14c, and includes the shaft part 34 formed on a base end side, the engagement parts 36 formed on a tip end part of the shaft part 34, and the conical part 38 formed on a tip end side of the engagement parts 36.

The shaft part 34 is a columnar part extending with a constant diameter from the piston abutting part 14c toward the tip end side in the axial direction. The shaft part 34 is formed to have a diameter substantially equal to or smaller than an inner diameter of the narrow part 28 in order to prevent the shaft part 34 from being caught by the narrow part 28 and the piston 22 from moving when being mounted to the piston 22. A diameter of the shaft part 34 is formed to be, for example, 1 mm to 5 mm. By providing the shaft part 34, the narrow part 28 extending with a constant diameter in the axial direction can be provided in the recess 24 of the piston 22, and strength of joint with the piston 22 in a pull-out direction is improved.

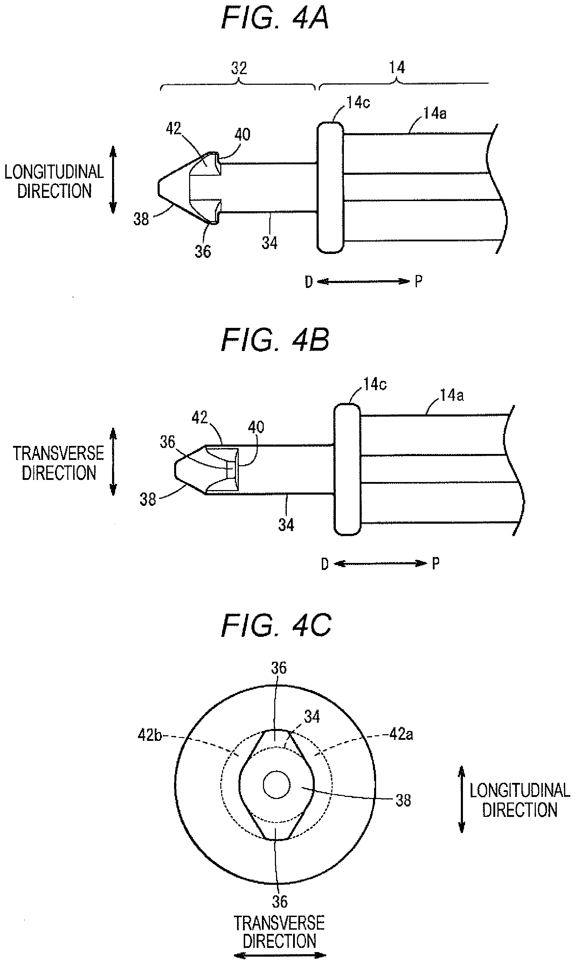

The engagement parts 36 are parts formed to increase in diameter outward in the radial direction from the shaft part 34. As illustrated in Fig. 4A, the engagement parts 36 are formed to project in an arrowhead shape, and a part perpendicular to a projection direction (β direction) of the engagement parts 36 are cut out by cut-out parts 42. As illustrated in Fig. 4C, the engagement parts 36 of the present embodiment are formed by cutting out a conical area from both sides in a width direction. That is, the engagement parts 36 are formed as parts remaining after cutting out, of an area increased in diameter conically, one side portion with a first cut-out part 42a and another side portion with a second cut-out part 42b. The first cut-out part 42a and the second cut-out part 42b are obtained by cutting out areas projecting beyond a diameter of the shaft part 34, and are formed in an arc shape along an outer periphery of a conical part as viewed from front. A pair of engagement parts 36 is formed to face each other while being spaced apart from each other by 180° in a circumferential direction.

It should be noted that the number of cut-out parts 42 is not limited to two as in the illustrated examples, and a plurality of cut-out parts may be provided at regular intervals in the circumferential direction. In addition, the number of the engagement parts 36 is not limited to two, and three or more engagement parts 36 may be provided.

As illustrated in Fig. 4B, as viewed from top, the engagement parts 36 are formed so as not to project beyond the shaft part 34 in the width direction. That is, the engagement parts 36 are formed to be flat in the width direction.

As illustrated in Fig. 4A, the engagement parts 36 are formed in a shape that gradually decreases in size toward the tip end until the outer diameter of the engagement parts 36 substantially coincides with an outer diameter of the base end of the conical part 38. With such a shape, when passing through the narrow part 28, the engagement parts 36 easily pass through the narrow part 28, and the mounting part 32 can be mounted more smoothly by the recess 24.

A plunger kit 15 and syringe kit 10 including the plunger rod 14 and piston 22 of the present embodiment are configured as described above, and operation thereof will be described below.

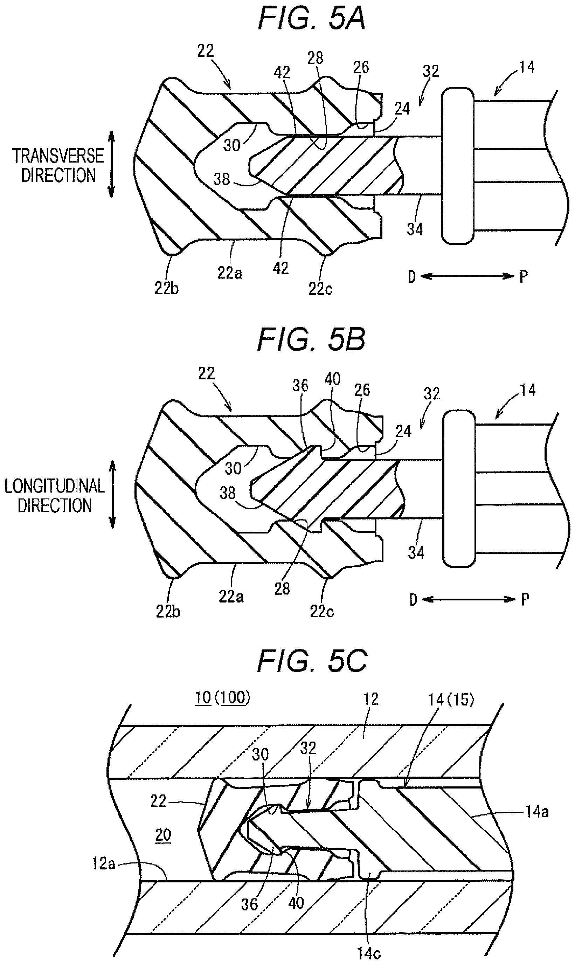

As illustrated in Fig. 5A, the plunger rod 14 is mounted to the piston 22 by inserting the mounting part 32 of the plunger rod 14 into the recess 24 of the piston 22. As illustrated in Fig. 5A, because the engagement parts 36 of the plunger rod 14 are provided with the cut-out parts 42 in the width direction, the engagement parts 36 are inserted in the width direction without deforming the narrow part 28 of the recess 24. Meanwhile, as illustrated in Fig. 5B, at portions where the engagement parts 36 project in the vertical direction, the engagement parts 36 moves forward while spreading out the narrow part 28. In addition, because the inner diameter of the narrow part 28 is larger than an outer diameter of the shaft part 34 of the plunger rod 14, the shaft part 34 of the plunger rod 14 does not spread out the narrow part 28.

In the present embodiment, because the engagement parts 36 increased in diameter from the shaft part 34 are cut out in the circumferential direction by the first cut-out part 42a and the second cut-out part 42b, a portion of the narrow part 28 where the engagement parts 36 spread out can be further reduced. As a result, the mounting part 32 including the engagement parts 36 can be mounted to the recess 24 with less pushing force. With this arrangement, the plunger rod 14 can be mounted to the piston 22 while preventing displacement of the piston 22 in the axial direction.

Thereafter, the plunger rod 14 is mounted to the piston 22, and as illustrated in Fig. 5C, the plunger kit 15, syringe kit 10, and prefilled syringe 100 of the present embodiment are completed. In the plunger kit 15, the engagement parts 36 are engaged with the narrow part 28 extending long in the axial direction inside the piston 22, and therefore, force required for deformation of the narrow part 28 is increased, enhancing joint strength in the pull-out direction, even in a case where load in the pull-out direction is input via the plunger rod 14. With this arrangement, even if force toward the base end direction acts on the plunger rod 14 during transportation or the like, the plunger rod 14 is less likely to fall off from the piston 22. In addition, because the inner diameter of the narrow part 28 is slightly larger than the outer diameter of the shaft part 34 of the plunger rod 14, an axis of the plunger rod 14 is less likely to incline with respect to an axis of the piston 22. Therefore, engagement between the engagement parts 36 of the plunger rod 14 and the narrow part 28 extending long in the axial direction inside the piston 22 is reliably maintained.

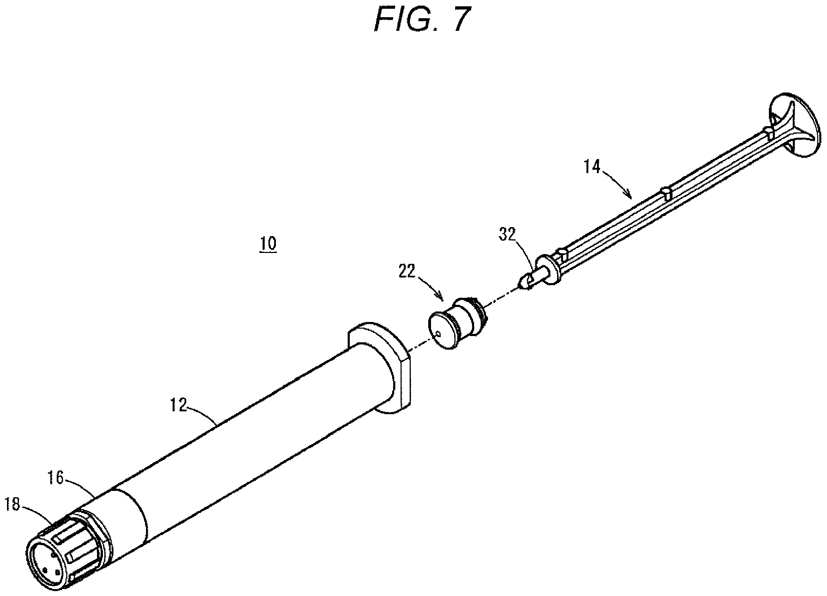

Although an example in which the syringe kit 10 is applied to the prefilled syringe 100 has been described above, the syringe kit 10 of the present embodiment is not limited thereto, and various aspects can be taken. For example, as illustrated in Fig. 7, the present invention may be provided in an aspect of including the syringe main body part 12 that does not store the liquid medicine 20, the piston 22 separated from the syringe main body part 12, and the plunger rod 14 separated from the syringe main body part 12 and the piston 22. In the syringe kit 10 in this aspect, the liquid medicine 20 is not injected in the syringe main body part 12, and the plunger kit 15 is assembled and mounted to the syringe main body part 12 immediately before the syringe kit 10 is used.

The plunger kit 15, syringe kit 10, and prefilled syringe 100 of the present embodiment have the following effects.

The plunger kit 15 of the present embodiment is a plunger kit 15 including the piston 22 to be slidably inserted into the liquid medicine storage chamber 12a of the syringe main body part 12, and the plunger rod 14 mountable to the piston 22, in which the plunger rod 14 has the rod main body 14a that extends in the axial direction and transmits drive force to the piston 22, and the mounting part 32 that projects from a tip end part of the rod main body 14a and is for mounting the plunger rod 14 to the piston 22, the mounting part 32 has the shaft part 34 that projects from the tip end part of the rod main body 14a to a tip end side with a substantially constant diameter, the engagement part 36 that projects from the tip end part of the shaft part 34 in the radial direction and forms the engagement surface 40 formed substantially perpendicular to the axial direction, and the conical part 38 that is disposed on a tip end side of the engagement part 36 and reduced in diameter in a tapered manner toward the tip end, the recess 24 is formed on the piston 22, the recess 24 having the opening part 26 opened in the base end direction, the narrow part 28 communicating with the tip end side of the opening part 26 and extending in the axial direction, and the storage part 30 communicating with a tip end side of the narrow part 28 and capable of storing the engagement part 36 and the conical part 38, the storage part 30 being formed in a hollow shape, the piston 22 has the first side wall part 30c forming the storage part 30 and the second side wall part 28a forming the narrow part 28 and thicker than the first side wall part 30c, length of the second side wall part 28a in the axis α1 direction is 10% to 40% of an entire length of the piston 22 in the axis α1 direction, and cut-out parts 42 obtained by cutting out a part in the circumferential direction are formed on the engagement part 36 of the plunger rod 14.

With the above-described configuration, as compared to a case where the engagement parts 36 are formed in an entire area in the circumferential direction, resistance of when the plunger rod 14 is mounted to the piston 22 is reduced, and the plunger rod 14 can be mounted to the piston 22 with small pushing force. With this arrangement, the plunger rod 14 can be mounted to the piston 22 without displacing the piston 22. In addition, because the piston 22 includes the second side wall part 28a formed to be thick and extend in the axial direction by a predetermined length, and the narrow part 28, the engagement parts 36 of the plunger rod 14 can be reliably held. With this arrangement, even if force in the base end direction acts on the plunger rod 14 during transportation or the like, the plunger rod 14 does not easily fall off from the piston 22.

In the above-described plunger kit 15, the narrow part 28 may extend with a substantially uniform diameter. With this arrangement, the engagement parts 36 can smoothly pass through the narrow part 28, and pressing force of when the plunger rod 14 is mounted can be reduced.

In the above-described plunger kit 15, the outer diameter of the shaft part 34 may be equal to or smaller than the inner diameter of the narrow part 28. With this arrangement, the shaft part 34 can be prevented from being caught by the narrow part 28, and the piston 22 can be prevented from moving.

In the above-described plunger kit 15, the piston 22 may have the ring-shaped protrusion 22c on an outer peripheral surface of the second side wall part 28a, the ring-shaped protrusion 22c abutting against the inner wall of the liquid medicine storage chamber 12a of the syringe main body part 12. With this arrangement, when the engagement parts 36 pass through the narrow part 28, the ring-shaped protrusion 22c is pressed against the inner wall of the liquid medicine storage chamber 12a as the narrow part 28 is spread out. As a result, the piston 22 is less likely to move, and movement of the piston 22 accompanying mounting of the plunger rod 14 can be prevented.

In the above-described plunger kit 15, the opening part 26 of the recess 24 may have the base end part 26a extending along the axis α1 direction by a predetermined length with an inner diameter substantially the same as the outer diameter of the engagement parts 36, and the tapered part 26b reduced in diameter from the base end part 26a toward the narrow part 28. With this arrangement, when the plunger rod 14 is inserted into the recess 24, the engagement parts 36 are guided by the base end part 26a, and the conical part 38 is guided by the tapered part 26b, by which an axis of the plunger rod 14 and the axis of the piston 22 coincide with each other. With this arrangement, the engagement parts 36 easily pass through the narrow part 28, and movement of the piston 22 can be prevented.

In the above-described plunger kit 15, the engagement parts 36 may have a shape that decreases in size toward the tip end until the outer diameter of the engagement parts 36 substantially coincides with the outer diameter of the conical part 38. With this arrangement, when the mounting part 32 is inserted into the recess 24, the engagement parts 36 guided by the conical part 38 can smoothly pass through the narrow part 28.

In the above-described plunger kit 15, a pair of engagement parts 36 may be provided spaced apart from each other by 180° in the circumferential direction.

In this case, a pair of cut-out parts 42a, 42b is formed across the shaft part 34, and a pair of engagement parts 36 is formed to project in an arrowhead shape. With such a configuration, when the plunger rod 14 is mounted, only the engagement parts 36, which thinly project, thrust the narrow part 28 of the piston 22, and the cut-out parts 42a, 42b can pass through the narrow part 28 without receiving resistance. With this arrangement, resistance of when the plunger rod 14 is mounted to the piston 22 is reduced, and the plunger rod 14 can be mounted to the piston 22 without displacing the piston 22.

In the above-described plunger kit 15, the cut-out parts 42a, 42b may be obtained by cutting out the engagement parts 36 within an area from the outer diameter of the engagement parts 36 to the outer diameter of the shaft part 34. With such a configuration, resistance of when the plunger rod 14 is mounted to the piston 22 is reduced, and the plunger rod 14 can be mounted to the piston 22 without displacing the piston 22.

In addition, the syringe kit 10 and prefilled syringe 100 of the present embodiment include the above-described plunger kit 15. With this arrangement, displacement of the piston 22 when the plunger rod 14 is mounted to the piston 22 can be prevented, and, after the plunger rod 14 is mounted, the plunger rod 14 can be prevented from coming off the piston 22 in the prefilled syringe 100 during transportation.

### (Second embodiment)

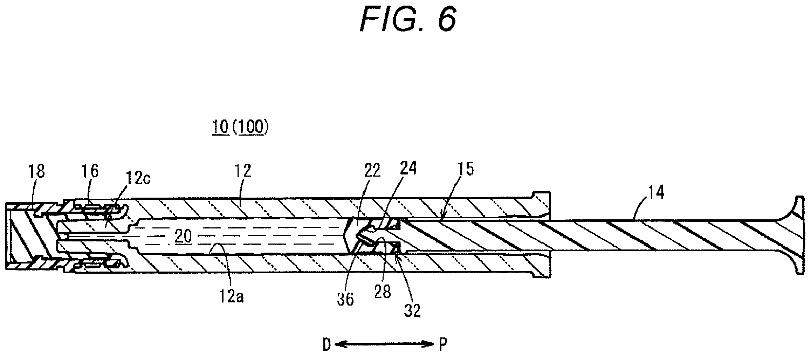

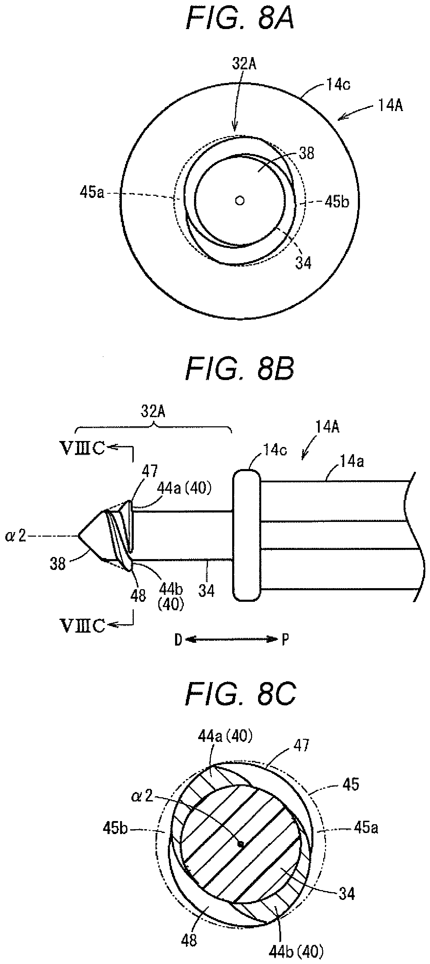

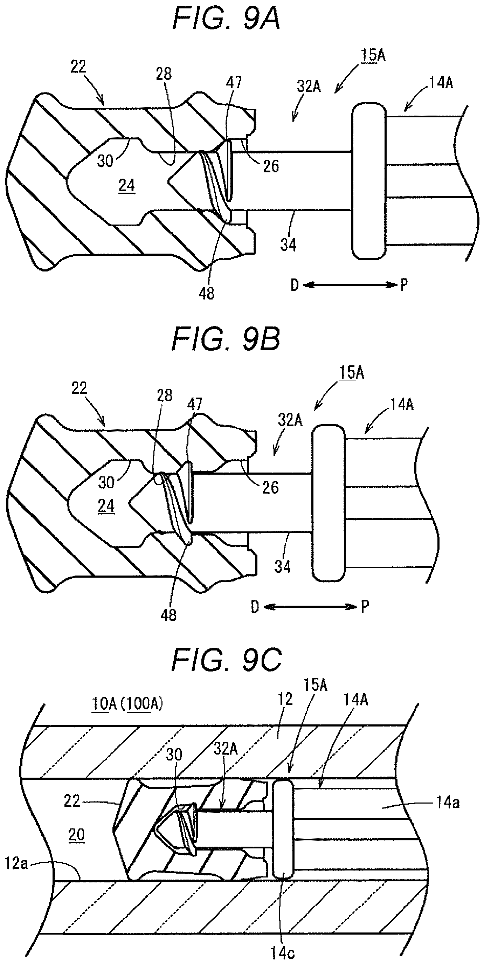

As illustrated in Fig. 9A, a plunger kit 15A of the present embodiment is different in a structure of a mounting part 32A of a plunger rod 14A from the plunger rod 14 described with reference to Figs. 1 to 6. It should be noted that, in Figs. 8A to 9C, a configuration similar to the configuration of the plunger kit 15 is denoted by the same reference sign, and detailed description thereof will be omitted.

As illustrated in Fig. 8A, engagement parts 47, 48 formed to be increased in diameter from a shaft part 34 is formed at the mounting part 32A of the plunger rod 14A of the present embodiment. These engagement parts 47, 48 are formed as parts partially cut out in a circumferential direction by a first cut-out part 45a and a second cut-out part 45b from an area 45 formed in a circular shape as viewed from front.

As illustrated in Fig. 8B, the engagement part 47 and the engagement part 48 are configured as thread ridges formed by being cut out by the first cut-out part 45a and second cut-out part 45b formed spirally, from a contour conically extending from a conical part 38 at a tip end toward a base end side. The first cut-out part 45a and the second cut-out part 45b are formed to be spaced apart from each other by 180° in the circumferential direction, and the engagement part 47 and the engagement part 48 form two thread ridges. It should be noted that the number of cut-out parts 45a, 45b is not limited to two, and may be three or more.

An engagement surface 44a and engagement surface 44b substantially perpendicular to an axial direction are formed at base ends of the engagement part 47 and engagement part 48, respectively. As illustrated by hatching in Fig. 8C, the engagement surfaces 44a, 44b are disposed to face each other across an axis α2. The engagement surfaces 44a, 44b abut against an abutting surface 30b1 of the piston 22, by which the engagement parts 47, 48 are engaged with the piston 22.

The plunger kit 15A of the present embodiment includes the plunger rod 14A described above, and operation thereof will be described below.

As illustrated in Figs. 9A and 9B, the plunger rod 14A of the present embodiment is screwed to be mounted while being moved forward from an opening part 26 of the piston 22 in the axial direction and rotated around an axis. The plunger rod 14A is mounted in such a manner that the engagement parts 47, 48 spread out a narrow part 28 of the piston 22. With this arrangement, as illustrated in Fig. 9C, the plunger kit 15A, syringe kit 10A, and prefilled syringe 100A of the present embodiment are assembled. In the present embodiment, force moving the plunger rod 14A forward is also dispersed in a rotation direction. Therefore, force pressing the piston 22 in the axial direction when the plunger rod 14A is mounted to the piston 22 decreases. With this arrangement, the plunger rod 14A can be mounted to the piston 22 while preventing movement of the piston 22.

The plunger kit 15A, syringe kit 10A, and prefilled syringe 100A of the present embodiment have the following effects.

In the plunger kit 15A of the present embodiment, the cut-out parts 45a, 45b are formed to extend spirally in the axial direction, and a plurality of thread ridges is formed on the engagement parts 47, 48. With this arrangement, the plunger rod 14A can be mounted to the piston 22 by operation that is a combination of pushing operation and screwing operation. With this arrangement, load of when the plunger rod 14A is mounted can be dispersed to load in the rotation direction, and therefore displacement of the piston 22 when the plunger rod 14A is mounted can be more effectively reduced.

In the above-described plunger kit 15A, a pair of engagement surfaces 44a, 44b may be provided spaced apart from each other by 180° in the circumferential direction. That is, it is possible to configure a double-start thread in which two engagement surfaces 44a and 44b serving as base ends of the thread ridges are formed. Such a configuration is preferable, because the engagement surfaces 44a, 44b to be engaged with the narrow part 28 of the piston 22 are increased in size, and therefore joint strength against load in a pull-out direction can be ensured.

In addition, the syringe kit 10A and prefilled syringe 100A of the present embodiment include the above-described plunger kit 15A. With this arrangement, displacement of the piston 22 can be prevented when the plunger rod 14A is mounted to the piston 22, and, after the plunger rod 14A is mounted, the plunger rod 14A can be prevented from coming off the piston 22 during transportation.

### (Third embodiment)

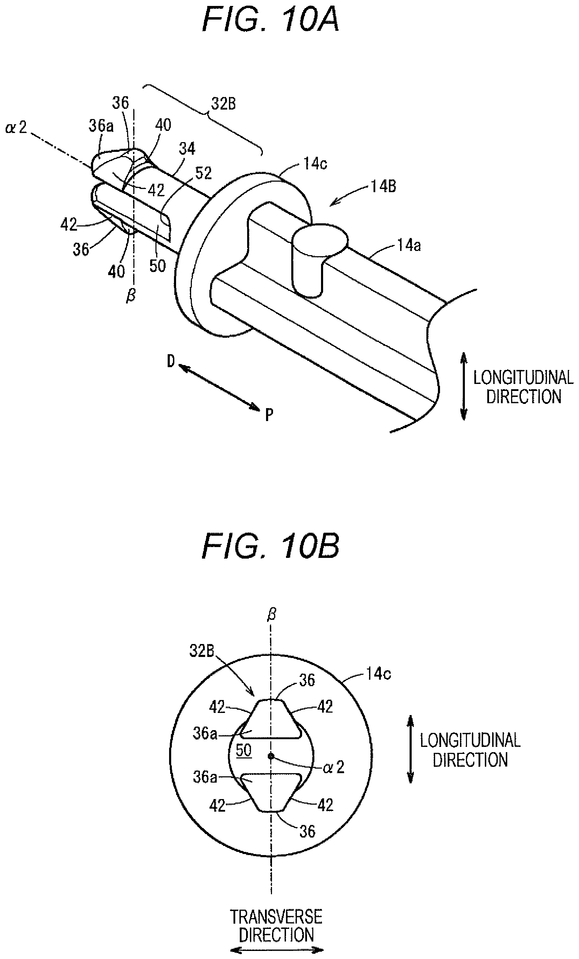

As illustrated in Fig. 10A, a plunger rod 14B of the present embodiment is different from the plunger rod 14 described with reference to Figs. 1 to 6 in that a slit structure 50 is provided on a mounting part 32B. Other parts are similar to the parts of the plunger rod 14, and similar configurations are denoted by the same reference signs, and detailed description thereof will be omitted.

As illustrated in Figs. 10A and 10B, the slit structure 50 is formed to pass between a pair of engagement parts 36 along an axis α2. The slit structure 50 is formed by cutting out a conical part 38 (refer to Fig. 3) at a tip end of the mounting part 32B, passing between the pair of engagement parts 36, and extending to a shaft part 34. An end part 52 is formed on a base end side of the slit structure 50, and the pair of engagement parts 36 is formed to extend in an arm shape from the end part 52. The engagement parts 36 are configured to be elastically deformable in a direction of an alternate long and short dash line β direction (longitudinal direction) inward starting from vicinity of the end part 52.

The plunger rod 14B of the present embodiment is configured as described above, and operation thereof will be described below.

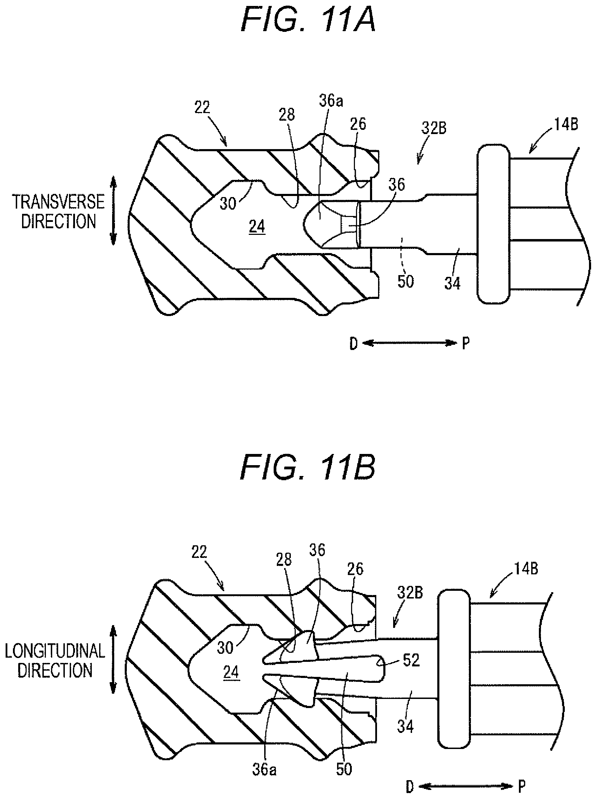

As illustrated in Fig. 11A, also in the plunger rod 14B of the present embodiment, the engagement parts 36 are cut out by cut-out parts 42, and therefore a dimension in a width direction is formed smaller than a dimension of a narrow part 28 of a piston 22. In addition, as illustrated in Fig. 11B, the pair of engagement parts 36 elastically deforms inward starting from the end part 52 of the slit structure 50. With this arrangement, a portion that thrusts the narrow part 28 when the plunger rod 14B is mounted to the piston 22 is reduced, and the plunger rod 14B can be pushed into the piston 22 with less resistance.

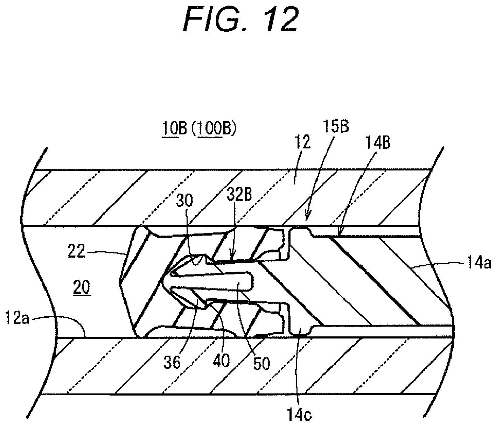

As illustrated in Fig. 12, the mounting part 32B expands outward by elastic restoring force after the engagement parts 36 pass through the narrow part 28, and an engagement surface 40 of each of the engagement parts 36 is engaged with the narrow part 28, by which joint strength against pulling out can be exerted. As described above, a plunger kit 15B, syringe kit 10B, and prefilled syringe 100B of the present embodiment are completed.

The plunger kit 15B, syringe kit 10B, and prefilled syringe 100B of the present embodiment have the following effects.

The plunger kit 15B of the present embodiment includes the slit structure 50 formed over the conical part 38, the engagement parts 36, and the shaft part 34, and the engagement parts 36 are elastically deformable inward. With such a configuration, the engagement parts 36 are elastically deformed inward when passing through the narrow part 28 of the piston 22, and therefore the plunger rod 14B can be mounted to the piston 22 with less resistance. As a result, the plunger rod 14B can be mounted to the piston 22 while preventing movement of the piston 22.

In addition, because the syringe kit 10B and prefilled syringe 100B of the present embodiment include the above-described plunger kit 15B, displacement of the piston 22 can be prevented when the plunger rod 14B is mounted to the piston 22. In addition, after the plunger rod 14B is mounted, the plunger rod 14B can be prevented from coming off the piston 22 during transportation.

### (Fourth embodiment)

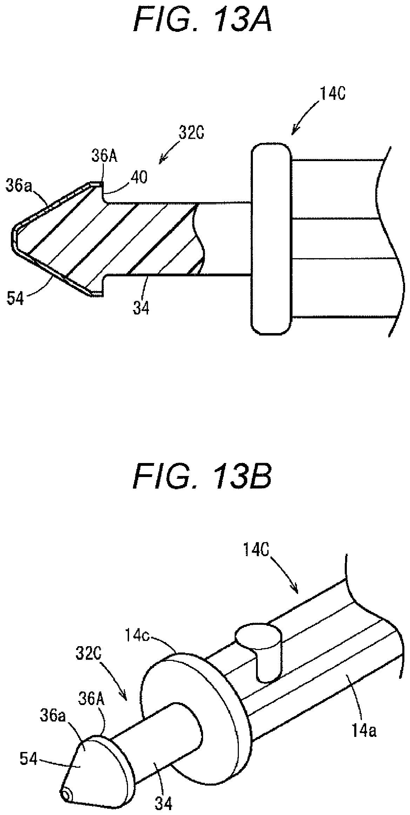

As illustrated in Fig. 13A, on a mounting part 32C in a plunger rod 14C of the present embodiment, coating 54 having lubricity is formed on a surface of a conical part 38 and an inclined surface 36a of engagement parts 36A. The coating 54 includes, for example, cross-linked silicone resin or fluororesin, and is configured to exert lubricity on a surface thereof. As illustrated in Fig. 13B, in the present embodiment, the engagement parts 36A may be conical engagement parts 36A not provided with cut-out parts 42 (refer to Fig. 3). It should be noted that the present embodiment is not limited to the illustrated examples, and the coating 54 may be formed on the inclined surface 36a of the engagement parts 36 on which the cut-out parts 42 illustrated in Figs. 4A to 4C are formed.

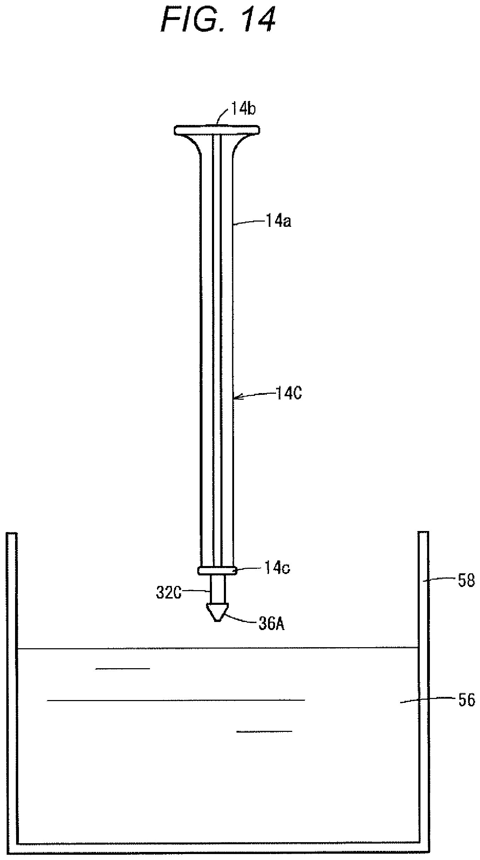

As illustrated in Fig. 14, the present embodiment can be formed by disposing he plunger rod 14C with the mounting part 32C thereof facing downward, and immersing the engagement parts 36A of the mounting part 32C in solution 56 of cross-linked silicone resin stored in a container 58.

The plunger rod 14C of the present embodiment has the following effects.

In the plunger rod 14C of the present embodiment, the coating 54 for improving sliding performance is formed on the conical part 38 and the engagement parts 36A. With this arrangement, resistance of when the plunger rod 14C is mounted to a piston 22 can be reduced, and movement of the piston 22 can be prevented. It should be noted that the conical part 38 and engagement parts 36A of the present embodiment may be those described with reference to Figs. 1 to 12.

### (Fifth embodiment)

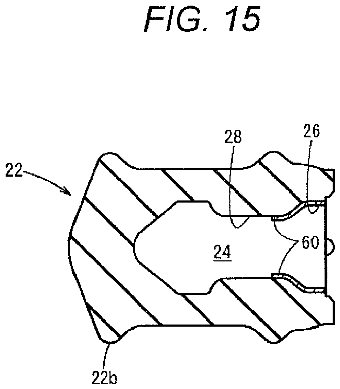

As illustrated in Fig. 15, in the present embodiment, coating 60 having lubricity is formed on a portion of a recess 24 of a piston 22. That is, the coating 60 including cross-linked silicone resin or fluororesin is formed on an opening part 26 on a base end side of the recess 24 and on a portion of a narrow part 28.

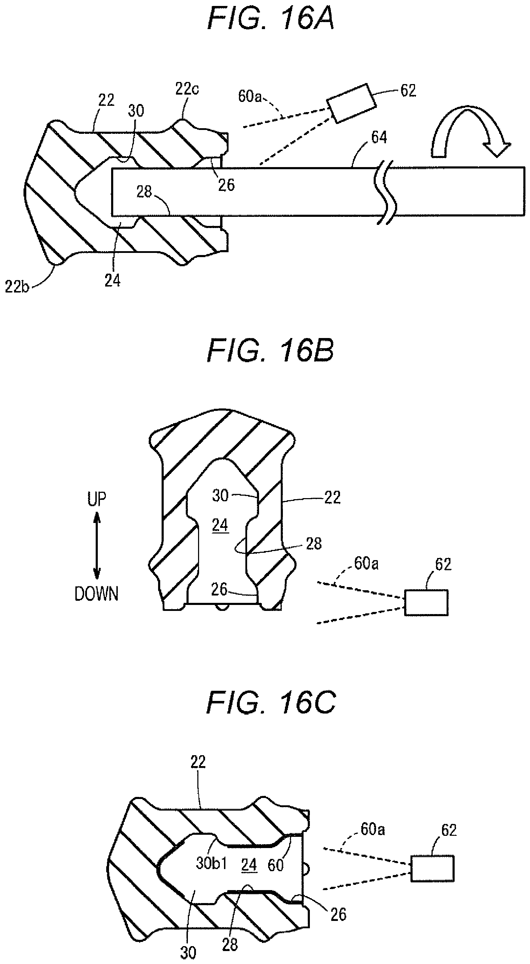

As illustrated in Fig. 16A for example, the piston 22 on which the coating 60 is formed can be formed by spraying a coating material 60a from an injection device 62 toward the recess 24 in a state where a jig 64 having a rod shape is inserted into the narrow part 28. The jig 64 adheres tightly to the narrow part 28, thereby functioning as a mask for preventing entry of the coating material 60a into a storage part 30. With this arrangement, the coating 60 (refer to Fig. 15) having lubricity can be formed only on the opening part 26 in vicinity of an inlet of the recess 24, and on a portion of the narrow part 28.

In addition, as illustrated in Fig. 16B for example, the piston 22 on which the coating 60 is formed may be formed by disposing the piston 22 with the opening part 26 of the recess 24 thereof facing vertically downward and spraying the coating material 60a from a side by the injection device 62. In this case, the coating 60 can be formed on the opening part 26 by portion of the injected coating material 60a going around the vicinity of the inlet of the recess 24.

In addition, as illustrated in Fig. 16C for example, the piston 22 on which the coating 60 is formed may be formed by disposing the injection device 62 to face the opening part 26 of the recess 24 of the piston 22 and spraying the coating material 60a to the recess 24. In this case, an abutting surface 30b1 that locks a plunger rod 14 (refer to Fig. 1) is a portion hidden with respect to the injection device 62, and therefore, the coating material 60a does not adhere to the abutting surface 30b1. Therefore, joint strength can be maintained in a pull-out direction.

As described above, in the present embodiment, the coating 60 for improving sliding performance is formed on the opening part 26 of the piston 22. With this arrangement, resistance of when the plunger rod 14 is mounted to the piston 22 can be reduced, and movement of the piston 22 can be prevented.

### (Sixth embodiment)

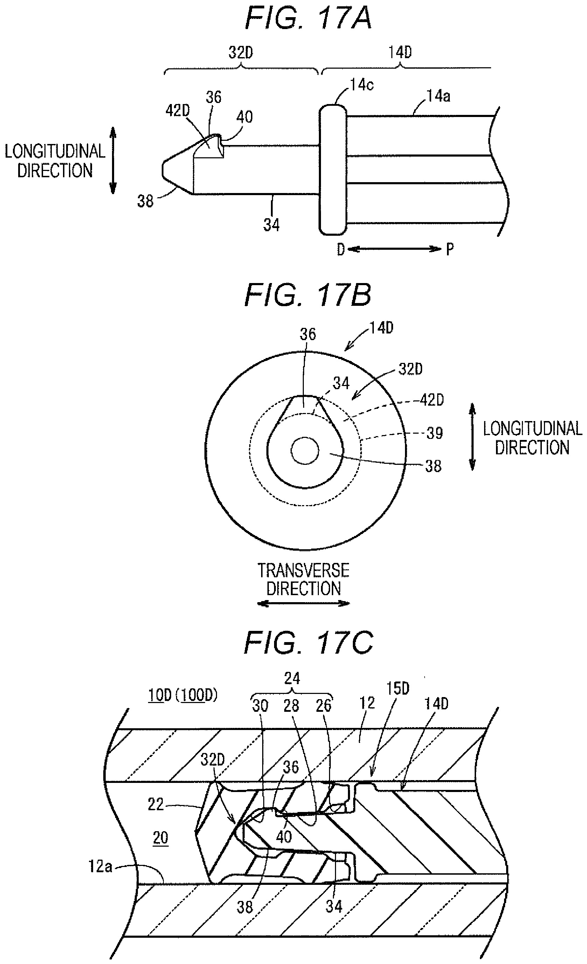

As illustrated in Fig. 17A, a plunger rod 14D of the present embodiment is different from the plunger rod 14 described with reference to Figs. 1 to 6 in that only one engagement part 36 of a mounting part 32D is formed to project. The engagement part 36 of the plunger rod 14D is a part formed to project from a shaft part 34, and as illustrated in Fig. 17B, is formed by cutting out a conical area 39 by a cut-out part 42D occupying most of an area in a circumferential direction. That is, the engagement part 36 is formed only at one position in the circumferential direction.

As illustrated in Fig. 17C, by inserting the mounting part 32D of the plunger rod 14D from a base end side of a piston 22, the plunger rod 14D is mounted to the piston 22, and a plunger kit 15D, syringe kit 10D, and prefilled syringe 100D of the present embodiment are assembled.

According to the plunger kit 15D, syringe kit 10D, and prefilled syringe 100D of the present embodiment, because most of an area in the engagement part 36 is cut out as the cut-out part 42D, when a narrow part 28 of the piston 22 is inserted, volume of the engagement part 36 that thrusts the narrow part 28 is reduced, and the plunger rod 14D can be mounted to the piston 22 with less pushing force. With this arrangement, the plunger rod 14D can be mounted without moving the piston 22. In addition, in the prefilled syringe 100D, the plunger rod 14D can be prevented from being pulled out from the piston 22 during transportation.

## Claims

1. A plunger kit (15, 15A, 15B) comprising a piston (22) to be slidably inserted into a liquid medicine storage chamber (12a) of a syringe main body part (12), and a plunger rod (14, 14A, 14B, 14C, 14D) mountable to the piston (22),
wherein the plunger rod (14, 14A, 14B, 14C, 14D) has a rod main body (14) that extends in an axial direction and transmits drive force to the piston (22), and a mounting part (32, 32A, 32B, 32C, 32E) that projects from a tip end of the rod main body (14) and is for mounting the plunger rod (14, 14A, 14B, 14C, 14D) to the piston (22),
the mounting part (32, 32A, 32B, 32C, 32E) has a shaft part (34) that projects from a tip end part of the rod main body (14) to a tip end side with a substantially constant diameter, an engagement part (36, 36A, 47, 48) that projects from a tip end part of the shaft part (34) in a radial direction and forms an engagement surface (40, 44a, 44b) formed substantially perpendicular to the axial direction, and a conical part (38) disposed on a tip end side of the engagement part (36, 36A, 47, 48) and reduced in diameter in a tapered manner toward a tip end,
a recess (24) is formed on the piston (22), the recess (24) having an opening part (26, 26a) opened in a base end direction, a narrow part (28) communicating with a tip end side of the opening part (26, 26a) and extending in the axial direction, and a storage part (30) communicating with a tip end side of the narrow part (28) and capable of storing the engagement part (36, 36A, 47, 48) and the conical part (38), the storage part (30) being formed in a hollow shape,
the piston (22) has a first side wall part (30c) forming the storage part (30), and a second side wall part (28a) forming the narrow part (28) and thicker than the first side wall part (30c),
length of the second side wall part (28a) in the axial direction is 10% to 40% of an entire length of the piston (22) in the axial direction, and
a cut-out part (42, 42a, 42b, 42D, 45a, 45b) obtained by cutting out a part in a circumferential direction is formed on the engagement part (36, 36A, 47, 48) of the plunger rod (14, 14A, 14B, 14C, 14D);
**characterized in that**
the plunger kit (15, 15A, 15B) further comprises a slit structure (50) formed over the conical part (38), the engagement part (36, 36A, 47, 48), and the shaft part (34), and wherein the engagement part (36, 36A, 47, 48) is elastically deformable inward.

2. The plunger kit (15, 15A, 15B) according to claim 1, wherein the narrow part (28) extends with a substantially uniform diameter.

3. The plunger kit (15, 15A, 15B) according to claim 1 or 2, wherein an outer diameter of the shaft part (34) is equal to or smaller than an inner diameter of the narrow part (28).

4. The plunger kit (15, 15A, 15B) according to any one of claims 1 to 3, wherein the piston (22) has a ring-shaped protrusion (22c) on an outer peripheral surface of the second side wall part (28a), the ring-shaped protrusion (22c) abutting against an inner wall of a liquid medicine storage chamber (12a) of the syringe main body part (12).

5. The plunger kit (15, 15A, 15B) according to any one of claims 1 to 4, wherein the opening part (26, 26a) of the recess (24) has a base end part (12b, 26a) extending by a predetermined length with an inner diameter substantially the same as an outer diameter of the engagement part (36, 36A, 47, 48) along the axial direction, and a tapered part (26b) reduced in diameter from the base end part (12b, 26a) toward the narrow part (28).

6. The plunger kit (15, 15A, 15B) according to any one of claims 1 to 5, wherein the engagement part (36, 36A, 47, 48) has a shape that decreases in size toward a tip end until the outer diameter of the engagement part (36, 36A, 47, 48) substantially coincides with an outer diameter of a base end of the conical part (38).

7. The plunger kit (15, 15A, 15B) according to any one of claims 1 to 6, wherein a pair of the engagement parts (36, 36A, 47, 48) is provided spaced apart from each other by 180° in the circumferential direction.

8. The plunger kit (15, 15A, 15B) according to any one of claims 1 to 7, wherein coating (54, 60) that improves sliding performance of the mounting part (32, 32A, 32B, 32C, 32E) of the plunger rod (14, 14A, 14B, 14C, 14D) with respect to the piston (22) is formed on an inner surface of the opening part (26, 26a) of the piston (22).

9. The plunger kit (15, 15A, 15B) according to any one of claims 1 to 8, wherein coating (54, 60) that improves sliding performance of the mounting part (32, 32A, 32B, 32C, 32E) of the plunger rod (14, 14A, 14B, 14C, 14D) with respect to the piston (22) is formed on an outer surface of the conical part (38) and an outer surface of the engagement part (36, 36A, 47, 48).

10. The plunger kit (15, 15A, 15B) according to any one of claims 1 to 9, wherein a pair of the cut-out parts is formed across the shaft part (34), and a pair of the engagement parts (36, 36A, 47, 48) is formed to project in an arrowhead shape.

11. The plunger kit (15, 15A, 15B) according to claim 10, wherein the cut-out part (42, 42a, 42b, 42D, 45a, 45b) is obtained by cutting out the engagement part (36, 36A, 47, 48) within an area (45, 39) from the outer diameter of the engagement part (36, 36A, 47, 48) to the outer diameter of the shaft part (34).

12. The plunger kit (15, 15A, 15B) according to any one of claims 1 to 9, wherein the cut-out part (42, 42a, 42b, 42D, 45a, 45b) is formed to extend spirally in the axial direction, and a thread ridge is formed on the engagement part (36, 36A, 47, 48).

13. A syringe kit (10, 10A, 10B, 10D) comprising:
a plunger kit (15, 15A, 15B) according to any one of claims 1 to 3; and
a syringe main body part (12) having a liquid medicine storage chamber (12a) that stores liquid medicine, a nozzle (12c) provided on a tip end of the liquid medicine storage chamber (12a), and a cap (18) that seals a tip end opening part (26, 26a) of the nozzle (12c),
wherein the piston (22) is slidable in the liquid medicine storage chamber (12a).

14. A prefilled syringe (100, 100A, 100B, 100D) comprising:
the syringe kit (10, 10A, 10B, 10D) according to claim 13; and
liquid medicine filled in the liquid medicine storage chamber (12a),
wherein the piston (22) is disposed so as to be slidable in the liquid medicine storage chamber (12a).

## Patentansprüche

1. Kolbenset (15, 15A, 15B), das einen Stempel (22), der gleitend in eine Speicherkammer (12a) für flüssige Medikamente eines Spritzenhauptkörperteils (12) eingesetzt wird, und eine Kolbenstange (14, 14A, 14B, 14C, 14D), die an dem Stempel (22) angebracht werden kann, umfasst,
wobei die Kolbenstange (14, 14A, 14B, 14C, 14D) einen Stangenhauptkörper (14) aufweist, der sich in einer axialen Richtung erstreckt und eine Antriebskraft auf den Stempel (22) überträgt, und ein Befestigungsteil (32, 32A, 32B, 32C, 32E), das von einem Spitzenende des Stangenhauptkörpers (14) hervorsteht und zur Befestigung der Kolbenstange (14, 14A, 14B, 14C, 14D) an dem Stempel (22) dient,
das Befestigungsteil (32, 32A, 32B, 32C, 32E) einen Schaftteil (34) aufweist, der von einem Spitzenendteil des Stangenhauptkörpers (14) zu einer Spitzenendseite mit einem im Wesentlichen konstanten Durchmesser hervorsteht, ein Eingriffsteil (36, 36A, 47, 48), das von einem Spitzenendteil des Schaftteils (34) in einer radialen Richtung hervorsteht und eine Eingriffsfläche (40, 44a, 44b) bildet, die im Wesentlichen senkrecht zu der axialen Richtung ausgebildet ist, und einen konischen Teil (38), der an einer Spitzenendseite des Eingriffsteils (36, 36A, 47, 48) angeordnet ist und der sich in einer verjüngenden Weise im Durchmesser in Richtung eines Spitzenendes verringert,
eine Ausnehmung (24) an dem Stempel (22) ausgebildet ist, wobei die Ausnehmung (24) einen Öffnungsteil (26, 26a), der in einer Basisendrichtung geöffnet ist, einen schmalen Teil (28), der mit einer Spitzenendseite des Öffnungsteils (26, 26a) in Verbindung steht und sich in der axialen Richtung erstreckt, und einen Speicherteil (30) aufweist, der mit einer Spitzenendseite des schmalen Teils (28) in Verbindung steht und in der Lage ist, den Eingriffsteil (36, 36A, 47, 48) und den konischen Teil (38) zu speichern, wobei der Speicherteil (30) in einer hohlen Form ausgebildet ist,
der Stempel (22) einen ersten Seitenwandteil (30c), der den Speicherteil (30) bildet, und einen zweiten Seitenwandteil (28a) aufweist, der den schmalen Teil (28) bildet und dicker als der erste Seitenwandteil (30c) ist,
die Länge des zweiten Seitenwandteils (28a) in der axialen Richtung 10 % bis 40 % einer gesamten Länge des Stempels (22) in der axialen Richtung beträgt, und ein ausgeschnittener Teil (42, 42a, 42b, 42D, 45a, 45b), der durch Ausschneiden eines Teils in einer Umfangsrichtung erhalten wird, an dem Eingriffsteil (36, 36A, 47, 48) der Kolbenstange (14, 14A, 14B, 14C, 14D) ausgebildet ist;
**dadurch gekennzeichnet, dass**
das Kolbenset (15, 15A, 15B) ferner eine Schlitzstruktur (50) umfasst, die über dem konischen Teil (38), dem Eingriffsteil (36, 36A, 47, 48) und dem Schaftteil (34) ausgebildet ist, und wobei der Eingriffsteil (36, 36A, 47, 48) nach innen elastisch verformbar ist.

2. Kolbenset (15, 15A, 15B) nach Anspruch 1, wobei sich der schmale Teil (28) mit einem im Wesentlichen gleichmäßigen Durchmesser erstreckt.

3. Kolbenset (15, 15A, 15B) nach Anspruch 1 oder 2, wobei ein Außendurchmesser des Schaftteils (34) gleich oder kleiner als ein Innendurchmesser des schmalen Teils (28) ist.

4. Kolbenset (15, 15A, 15B) nach einem der Ansprüche 1 bis 3, wobei der Stempel (22) einen ringförmigen Vorsprung (22C) an einer äußeren Umfangsfläche des zweiten Seitenwandteils (28a) aufweist, wobei der ringförmige Vorsprung (22C) gegen eine Innenwand einer Speicherkammer (12a) für flüssige Medikamente des Spritzenhauptkörperteils (12) stößt.

5. Kolbenset (15, 15A, 15B) nach einem der Ansprüche 1 bis 4, wobei der Öffnungsteil (26, 26a) der Ausnehmung (24) einen Basisendteil (12b, 26a), der sich über eine vorbestimmte Länge mit einem Innendurchmesser erstreckt, welcher im Wesentlichen der gleiche ist wie ein Außendurchmesser des Eingriffsteils (36, 36A, 47, 48) entlang der axialen Richtung, und einen sich verjüngenden Teil (26b) aufweist, der im Durchmesser von dem Basisendteil (12b, 26a) in Richtung des schmalen Teils (28) hin abnimmt.

6. Kolbenset (15, 15A, 15B) nach einem der Ansprüche 1 bis 5, wobei der Eingriffsteil (36, 36A, 47, 48) eine Form aufweist, die in der Richtung eines Spitzenendes in der Größe abnimmt, bis der Außendurchmesser des Eingriffsteils (36, 36A, 47, 48) im Wesentlichen mit einem Außendurchmesser eines Basisendes des konischen Teils (38) übereinstimmt.

7. Kolbenset (15, 15A, 15B) nach einem der Ansprüche 1 bis 6, wobei ein Paar der Eingriffsteile (36, 36A, 47, 48) in der Umfangsrichtung um 180° voneinander beabstandet vorgesehen ist.

8. Kolbenset (15, 15A, 15B) nach einem der Ansprüche 1 bis 7, wobei eine Beschichtung (54, 60), welche die Gleiteigenschaft des Befestigungsteils (32, 32A, 32B, 32C, 32E) der Kolbenstange (14, 14A, 14B, 14C, 14D) in Bezug auf den Stempel (22) verbessert, auf einer Innenfläche des Öffnungsteils (26, 26a) des Stempels (22) ausgebildet ist.

9. Kolbenset (15, 15A, 15B) nach einem der Ansprüche 1 bis 8, wobei eine Beschichtung (54, 60), welche die Gleiteigenschaft des Befestigungsteils (32, 32A, 32B, 32C, 32E) der Kolbenstange (14, 14A, 14B, 14C, 14D) in Bezug auf den Stempel (22) verbessert, auf einer Innenfläche des Öffnungsteils (38) des Stempels (36, 36A, 47, 28) ausgebildet ist.

10. Kolbenset (15, 15A, 15B) nach einem der Ansprüche 1 bis 9, wobei ein Paar der ausgeschnittenen Teile quer über dem Schaftteil (34) ausgebildet ist und ein Paar der Eingriffsteile (36, 36A, 47, 48) so ausgebildet ist, dass es pfeilförmig vorsteht.

11. Kolbenset (15, 15A, 15B) nach Anspruch 10, wobei der ausgeschnittene Teil (42, 42a, 42b, 42D, 45a, 45b) durch Ausschneiden des Eingriffsteils (36, 36A, 47, 48) innerhalb eines Bereichs (45, 39) von dem Außendurchmesser des Eingriffsteils (36, 36A, 47, 48) zu dem Außendurchmesser des Schaftteils (34) erhalten wird.

12. Kolbenset (15, 15A, 15B) nach einem der Ansprüche 1 bis 9, wobei der ausgeschnittene Teil (42, 42a, 42b, 42D, 45a, 45b) so ausgebildet ist, dass er sich spiralförmig in der axialen Richtung erstreckt, und ein Gewinderücken an dem Eingriffsteil (36, 36A, 47, 48) ausgebildet ist.

13. Spritzenset (10, 10A, 10B, 10D), umfassend:
ein Kolbenset (15, 15A, 15B) nach einem der Ansprüche 1 bis 3; und
ein Spritzenhauptkörperteil (12), das eine Speicherkammer (12a) für flüssige Medikamente, die flüssige Medikamente speichert, eine Düse (12c), die an einem Spitzenende der Speicherkammer (12a) für flüssige Medikamente vorgesehen ist, und eine Kappe (18) aufweist, die ein Öffnungsteil (26, 26a) des Spitzenendes der Düse (12c) abdichtet,
wobei der Stempel (22) in der Speicherkammer (12a) für flüssige Medikamente verschiebbar ist.

14. Vorgefüllte Spritze (100, 100A, 100B, 100D), umfassend:
das Spritzenset (10, 10A, 10B, 10D) nach Anspruch 13; und
ein flüssiges Medikament, das in die Speicherkammer (12a) für flüssige Medikamente eingefüllt ist,
wobei der Stempel (22) so angeordnet ist, dass er in der Speicherkammer (12a) für flüssige Medikamente verschiebbar ist.

## Revendications

1. Kit de plongeur (15, 15A, 15B) comprenant un piston (22) à insérer de manière coulissante dans une chambre de stockage de médicament liquide (12a) d'une partie de corps principal de seringue (12), et une tige de plongeur (14, 14A, 14B, 14C, 14D) pouvant être montée sur le piston (22), dans lequel la tige de plongeur (14, 14A, 14B, 14C, 14D) a un corps principal de tige (14) qui s'étend dans une direction axiale et transmet une force d'entraînement au piston (22), et une partie de montage (32, 32A, 32B, 32C, 32E) qui fait saillie à partir d'une extrémité d'embout du corps principal de tige (14) et sert à monter la tige de plongeur (14, 14A, 14B, 14C, 14D) sur le piston (22),
la partie de montage (32, 32A, 32B, 32C, 32E) présente une partie d'arbre (34) qui fait saillie à partir d'une partie d'extrémité d'embout du corps principal de tige (14) jusqu'à un côté extrémité d'embout avec un diamètre sensiblement constant, une partie d'engagement (36, 36A, 47, 48) qui fait saillie à partir d'une partie d'extrémité d'embout de la partie d'arbre (34) dans une direction radiale et forme une surface d'engagement (40, 44a, 44b) formée sensiblement perpendiculairement à la direction axiale, et une partie effilée (38) disposée sur un côté extrémité d'embout de la partie d'engagement (36, 36A, 47, 48) et réduite en diamètre de manière effilée vers une extrémité d'embout,
un renfoncement (24) est formé sur le piston (22), le renfoncement (24) ayant une partie d'ouverture (26, 26a) ouverte dans une direction d'extrémité de base, une partie étroite (28) communiquant avec un côté extrémité d'embout de la partie d'ouverture (26, 26a) et s'étendant dans la direction axiale, et une partie de stockage (30) communiquant avec un côté extrémité d'embout de la partie étroite (28) et capable de stocker la partie d'engagement (36, 36A, 47, 48) et la partie effilée (38), la partie de stockage (30) étant formée en une forme creuse, le piston (22) a une première partie de paroi latérale (30c) formant la partie de stockage (30), et une deuxième partie de paroi latérale (28a) formant la partie étroite (28) et plus épaisse que la première partie de paroi latérale (30c), une longueur de la deuxième partie de paroi latérale (28a) dans la direction axiale représente 10 % à 40 % d'une longueur totale du piston (22) dans la direction axiale, et
une partie découpée (42, 42a, 42b, 42D, 45a, 45b) obtenue en découpant une partie dans une direction circonférentielle est formée sur la partie d'engagement (36, 36A, 47, 48) de la tige de plongeur (14, 14A, 14B, 14C, 14D) ;
**caractérisé en ce que**
le kit de plongeur (15, 15A, 15B) comprend en outre une structure de fente (50) formée sur la partie effilée (38), la partie d'engagement (36, 36A, 47, 48) et la partie d'arbre (34), et dans lequel la partie d'engagement (36, 36A, 47, 48) est élastiquement déformable vers l'intérieur.

2. Kit de plongeur (15, 15A, 15B) selon la revendication 1, dans lequel la partie étroite (28) s'étend avec un diamètre sensiblement uniforme.

3. Kit de plongeur (15, 15A, 15B) selon la revendication 1 ou 2, dans lequel un diamètre externe de la partie d'arbre (34) est égal ou inférieur à un diamètre interne de la partie étroite (28).

4. Kit de plongeur (15, 15A, 15B) selon l'une quelconque des revendications 1 à 3, dans lequel le piston (22) présente une protubérance en forme d'anneau (22c) sur une surface périphérique externe de la deuxième partie de paroi latérale (28a), la protubérance en forme d'anneau (22c) venant en butée contre une paroi interne d'une chambre de stockage de médicament liquide (12a) de la partie de corps principal de seringue (12).

5. Kit de plongeur (15, 15A, 15B) selon l'une quelconque des revendications 1 à 4, dans lequel la partie d'ouverture (26, 26a) du renfoncement (24) a une partie d'extrémité de base (12b, 26a) s'étendant sur une longueur prédéterminée avec un diamètre interne sensiblement identique à un diamètre externe de la partie d'engagement (36, 36A, 47, 48) suivant la direction axiale, et une partie effilée (26b) réduite en diamètre à partir de la partie d'extrémité de base (12b, 26a) vers la partie étroite (28).

6. Kit de plongeur (15, 15A, 15B) selon l'une quelconque des revendications 1 à 5, dans lequel la partie d'engagement (36, 36A, 47, 48) a une forme qui diminue en taille vers une extrémité d'embout jusqu'à ce que le diamètre externe de la partie d'engagement (36, 36A, 47, 48) coïncide sensiblement avec un diamètre externe d'une extrémité de base de la partie effilée (38).

7. Kit de plongeur (15, 15A, 15B) selon l'une quelconque des revendications 1 à 6, dans lequel une paire des parties d'engagement (36, 36A, 47, 48) est prévue espacée l'une de l'autre de 180° dans la direction circonférentielle.

8. Kit de plongeur (15, 15A, 15B) selon l'une quelconque des revendications 1 à 7, dans lequel un revêtement (54, 60) qui améliore les performances de coulissement de la partie de montage (32, 32A, 32B, 32C, 32E) de la tige de plongeur (14, 14A, 14B, 14C, 14D) par rapport au piston (22) est formé sur une surface interne de la partie d'ouverture (26, 26a) du piston (22).

9. Kit de plongeur (15, 15A, 15B) selon l'une quelconque des revendications 1 à 8, dans lequel un revêtement (54, 60) qui améliore les performances de coulissement de la partie de montage (32, 32A, 32B, 32C, 32E) de la tige de plongeur (14, 14A, 14B, 14C, 14D) par rapport au piston (22) est formé sur une surface externe de la partie effilée (38) et sur une surface externe de la partie d'engagement (36, 36A, 47, 48).

10. Kit de plongeur (15, 15A, 15B) selon l'une quelconque des revendications 1 à 9, dans lequel une paire des parties découpées est formée à travers la partie d'arbre (34), et une paire des parties d'engagement (36, 36A, 47, 48) est formée pour faire saillie en forme de pointe de flèche.

11. Kit de plongeur (15, 15A, 15B) selon la revendication 10, dans lequel la partie découpée (42, 42a, 42b, 42D, 45a, 45b) est obtenue en découpant la partie d'engagement (36, 36A, 47, 48) au sein d'une zone (45, 39) allant du diamètre externe de la partie d'engagement (36, 36A, 47, 48) au diamètre externe de la partie d'arbre (34) .

12. Kit de plongeur (15, 15A, 15B) selon l'une quelconque des revendications 1 à 9, dans lequel la partie découpée (42, 42a, 42b, 42D, 45a, 45b) est formée pour s'étendre en spirale dans la direction axiale, et une crête de filetage est formée sur la partie d'engagement (36, 36A, 47, 48).

13. Kit de seringue (10, 10A, 10B, 10D) comprenant :
un kit de plongeur (15, 15A, 15B) selon l'une quelconque des revendications 1 à 3 ; et
une partie de corps principal de seringue (12) présentant une chambre de stockage de médicament liquide (12a) qui stocke un médicament liquide, une buse (12c) prévue sur une extrémité d'embout de la chambre de stockage de médicament liquide (12a), et un capuchon (18) qui scelle une partie d'ouverture d'extrémité d'embout (26, 26a) de la buse (12c),
dans lequel le piston (22) peut coulisser dans la chambre de stockage de médicament liquide (12a).

14. Seringue préremplie (100, 100A, 100B, 100D) comprenant :
le kit de seringue (10, 10A, 10B, 10D) selon la revendication 13 ; et
un médicament liquide remplissant la chambre de stockage de médicament liquide (12a),
dans laquelle le piston (22) est disposé de façon à pouvoir coulisser dans la chambre de stockage de médicament liquide (12a).
